# EUROPEAN PATENT SPECIFICATION

(11) **EP 2 522 655 B1**
(45) Date of publication and mention of the grant of the patent: **19.07.2017**
(21) Application number: 10841903.7
(22) Date of filing: 18.03.2010
(51) Int. Cl.: C07C 403/24, C09B 61/00

(54) **PREPARING METHOD FOR XANTHOPHYLL CRYSTALS WITH HIGHER CONTENT OF ZEAXANTHIN FROM PLANT OLEORESIN**
HERSTELLUNGSVERFAHREN FÜR XANTHOPHYLLKRISTALLE MIT HOHEM ANTEIL AN ZEAXANTHIN AUS PFLANZLICHEM OLEORESIN
PROCÉDÉ DE PRÉPARATION DE CRISTAUX DE XANTHOPHYLLE DE TENEUR SUPÉRIEURE EN ZÉAXANTHINE D'OLÉORÉSINE VÉGÉTALE

(30) Priority: 07.01.2010 CN 201010039569
(43) Date of publication of application: 14.11.2012
(73) Proprietor: ZheJiang Medicine Co., Ltd. Xinchang Pharmaceutical Factory, Xinchang Zhejiang 312500 (CN)
(72) Inventor: XU, Xinde, Shaoxing Zhejiang 312500 (CN); SHAO, Bin, Shaoxing Zhejiang 312500 (CN); CHAO, Hongjuan, Shaoxing Zhejiang 31250 (CN); LAO, Xuejun, Shaoxing Zhejiang 312500 (CN); SUN, Xiaoxia, Shaoxing Zhejiang 312500 (CN)
(74) Representative: ProI European Patent Attorneys
(86) International application number: PCT/CN2010/071061
(87) International publication number: WO 2011/082560

(56) References cited:
- CN-A- 101 124 944
- US-A- 5 780 693
- US-A- 5 973 211
- US-A1- 2007 265 351
- US-A1- 2009 118 379
- US-B1- 6 504 067
- US-B2- 7 271 298
- US-B2- 7 485 738

## Description

### TECHNICAL FIELD

The present invention relates to the preparation of carotenoids, and in particular, relates to a method for the preparation of lutein crystals with a higher content of zeaxanthin from a plant oleoresin (particularly a marigold oleoresin).

### BACKGROUND

The carotenoid is a kind of widely naturally existed material with a color from yellow to red. In different fruits and vegetables, there exist different kinds of carotenoids, for example, beta-carotene in carrots, lutein in marigold flowers, and zeaxanthin in strawberries, lycopene in tomatoes. Chili class plants are quite rich in capsanthin and capsochrome. Some fruits and vegetables with dark color, the yolk, some carapaces fish, some birds, seaweed and some bacterium are quite rich in carotenoid, and in some of these materials, the content of lutein is also quite high.

In recent years, some animal and human experiments have proved the beneficial effect of carotenoid. In general, the carotenoid may be categorized into two subclasses, and they are the lutein class or carotenoids containing oxygen with a relatively strong polarity, such as lutein, zeaxanthin, astaxanthin, and the like, as well as nonpolar carbon and hydrogen class carotene, such as beta-carotene, lycopene, and the like. Both of the two subclasses of carotenoids contain at least nine conjugated double bonds. The conjugated double bonds not only endow the carotenoids certain color, but also assign the carotenoids quite a strong antioxidation effect in prevention and treatment of diseases, and they can also prevent cancer, arteriosclerosis, cataract, spot degradation, as well as other diseases. In addition, because of the capabilities of carotenoids in eliminating highly efficiently reactive oxygen free radicals and preventing generation of the free radicals, these carotenoids can limit the oxidizing destruction effect caused by the free radicals.

In all the carotenoids, because their potential capability to prevent one kind of age related macular degenerations (ARMD), lutein and zeaxanthin are drawing more and more attention from the scientists and the public. The lutein and zeaxanthin are the only carotenoids present within the spot area (macular degeneration) of human being's retina, and this area is closely related to the visual sensitivity of human beings (Bone et al. Invest. Ophthamal. Vis. Sci. 34:2033-2040, 1993). The risk of getting age related macular degeneration may be reduced by 43% when the fruits and vegetables that are rich in lutein and zeaxanthin are often eaten (Seddon et al. J. Am. Med. Assoc. 272:1413-1420, 1994), and the metabolic pathway of these compounds for preventing age related macular degeneration has been clear at present. The Food and Drug Administration also considers lutein and zeaxanthin as "generally recognized as safe" (GRAS). Therefore, these carotenoids can be used separately or together with other materials as nutritional supplements and food coloring agents, as well as be used clinically for preventing age related macular degeneration and cancer and the like.

The structural formulas of lutein and zeaxanthin are as follows respectively. They are isomers, and the only difference between zeaxanthin and lutein is the position of double bonds on one (not two) end ring. To be specific, the position of the double bonds on the two end rings of the former is symmetrical, whereas that of the latter is asymmetrical, i.e., the entire straight chain parts of the lutein and zeaxanthin of each molecule are both a conjugation structure which has alternant double bonds and a single bond. In the molecules of zeaxanthin, the conjugation structure extends to the first bond on the two end rings, whereas the conjugation extent of lutein is lower, because double bonds thereof on one of its end rings do not form the correct arrangement of complete conjugation structure, which is the difference of molecular structure that results in a certain difference between lutein and zeaxanthin in terms of functionality and property. 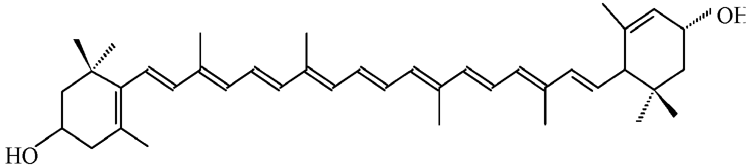 Lutein (molecular formula: C₄₀H₅₆O₂, molecular weight: 568.85) 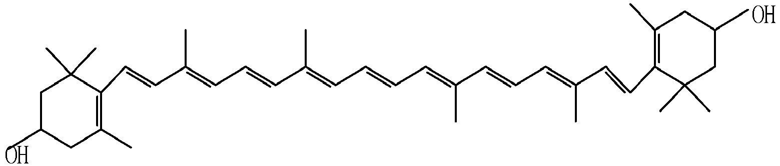 Zeaxanthin (molecular formula: C₄₀H₅₆O₂, molecular weight: 568.85)

As natural pigments, lutein and zeaxanthin exist widely in nature, they mainly exist in higher plants, algae, fishes, shells and bacteria, and they exist in the forms of ester in the body of living beings. Among theses living beings, the marigold flower is good source of lutein and zeaxanthin, there is generally 2 g lutein in 100 g of fresh marigold flowers, and among these xanthophyll substances, the major part is lutein which accounts for more than 90%, and the rest are zeaxanthin and a few other carotenoids. Like the marigold flowers, in other higher-class plant and algae sources, the proportion of lutein is much more then that of zeaxanthin, but the conment of zeaxanthin in corns is higher than that of lutein. In addition, from the molecular structure it can find that there are steroisomers in both lutein and zeaxanthin. With different sources of lutein and zeaxanthin, and the stereoisomerism compositions are also different. For example, (3R,3'R,6'R)-lutein or (3R,3'R)-zeaxanthin is the major component in plant resources, but lutein exists in forms of (3R,3'R,6'R)-, (3R,3'R,6'S)-, and (3R,3'S,6'S)- in animal resources, such as in fishes and shells.

Since chemical synthesis of the lutein involves many steps of reactions, a lot of time and manpower may be consumed, and the cost is very high. A quite economic and simpler way to obtain lutein in a large scale is to obtain the lutein from natural resources through extraction, separation and purification.

Many vegetables and fruits, such as spinaches, broccolis, cabbages and corns, etc, are quite rich in xanthophylls, but marigold flowers and calendulas are the richest sources of xanthophylls, of course, there also exists other carotenoid in these plants. The xanthophylls in plants usually exist in forms of single ester and double ester which is formed through esterification between the lutein and some C12-C18 long-chain aliphatic acids, such as myristic acid, linoleic acid and palmitic acid.

Generally, the lutein ester is extracted from plants, preferentially from marigold flowers, calendulas and other dark green vegetables, by using an organic solvent. In addition, the organic solvent may be simply removed by separation. The marigold extract (marigold oleoresin) is a very good lutein ester source, and the other carotenoids contain less lutein ester. After being hydrolyzed under alkaline conditions, the lutein crystals will be dissociated, and the lutein crystals may be further purified after the fatty acid salt resulted from the saponification process is washed off.

Meanwhile, as described above, since there exists one more conjugated double bond in the molecular structure, which makes the zeaxanthin have stronger antioxidant activity than the lutein, and the zeaxanthin achieves a more important function for the health of human eyes. In fact, some researches in the middle to late 1980s had also proved that they mainly were zeaxanthin in the small area at the center of human eyes' macula lutea. They leave the concave concentrically and get close to the circum of the macula lutea, so the quantity of zeaxanthin gradually becomes less, and the quantiy of lutein gradually becomes more. At the circum of the macula lutea, lutein is the main xanthophylls.

This also can be found from the change of the proportion between lutein and zeaxanthin in different parts of natural and human tissues, in the marigold flowers, the raw materials of xanthophylls, the proportion between lutein and zeaxanthin is about 10-12:1, their proportion is about 3-5:1 in human blood, and the proportion is 3:1 at the circum of retina macula lutea, but at the center of macula lutea the proportion is completely opposite, and it is 1:3. The recent researches have found that a stereoisomer of zeaxanthin, (3R, 3'S, meso)-zeaxanthin, at the central area of macula lutea, took quite a lot proportion, and the proportion will be less when closer to the periphery. More and more evidences have proved that the meso-zeaxanthin in macula lutea are production of isomerization through lutein, because it almost can not detect this kind of isomers of zeaxanthin in nature, human blood and other human tissues.

Changes of the distribution proportion of lutein and zeaxanthin in different positions in human eyes just prove the important and unique effects of zeaxanthin, in particular, the meso-zeaxanthin, for the health of human eyes. In practice, many human-based experiments also have proved that a complex use of lutein and zeaxanthin achieves a better effect. Therefore, most the eye care lutein-containing products on the market are generally added with a specific amount of extra zeaxanthin, i.e., lutein and zeaxanthin are respectively added according to a specific proportion in the applied formulation. However, the respective addition of lutein and zeaxanthin inevitably brings many unnecessary troubles for the purchase of starting materials, operations of production process and product quality control. If some parts of the lutein are isomerized into zeaxanthin during the process of production of lutein, it may be ensured that the final product contains both lutein and zeaxanthin in a desired proportion. In this way, during the product application, one carotenoid formulation having both lutein and zeaxanthin only needs to be added, which will brings great convenience for the subsequent product application process.

At present, there also are some patents and arts that refer to the method to separate lutein crystals in large scale from the peaberry of marigold or the method to gain zeaxanthin crystals through the isomerization of lutein. The targets of these essays generally are to obtain quite pure crystal forms of lutein or zeaxanthin, and many separation steps are involved.

In U.S.Pat.No.5,382,714, lutein is separated and purified by washing the saponified marigold oleoresin under a quite low temperature and crystallizing the mixed solvent under a low temperature. The purification process not only is time-consuming, but also uses chlorinated organic solvents, and thus the product obtained is not suitable for use in food and medicines. The ratio of lutein to zeaxanthin in the product does not change much compared with the raw materials.

In U.S.Pat.No.5,648,564, a method for separating lutein crystals is disclosed, wherein the method soap-dissolves lutein diester-containing the propylene glycol solution of marigold oleoresins first, and then recrystallization is performed. There are also several defects in this process. Firstly, since the viscosity of propylene glycol is quite high, a quite high temperature is required during the subsequent saponification process; the entire system needs to be kept at a temperature higher than 70°C for about 10 hours, which is obviously disadvantageous for the stability of lutein; the cis-trans isomers of lutein may also change; furthermore, the following separation processes, such as centrifugation and filtration, are also quite difficult. Secondly, the collection rate of lutein is quite low, which is about 59%, and the lutein content in the products is also not high.

In U.S.Pat.No.6,262,284, a method to use tetrahydrofuran to extract and soap-dissolve carotenoid from marigold dry flowers is disclosed, wherein a plenty of organic solvents are used in the process, and these solvents are unfavorable to the stability of lutein, and may result in deterioration due to overoxidation.

In U.S.Pat.No.6.329,557, a method for extracting lutein crystals from marigold oleoresins in large industrial scale is disclosed. The defect of the process is to use plenty of organic solvents, such as n-hexanes, ketones or the like, and these solvents are not suitable for use in food.

In U.S.Pat.No.6, 380,442, a method for separate carotenoid from plants is disclosed, wherein the method is also not attractive for industrial production, because a lot of water (at least 30 times the raw materials) is used during the production process, and the operation is quite difficult.

In U.S.Pat.No.6, 743,953, lutein is separated from marigold oleoresins and purified by using organic solvents. In this process, several organic solvents, such as isopropanol, ethyl acetate, n-hexane, acetone, methanol and the like, are used, the operations are complicated, the organic solvent consumption is quite large, and the yield is low. Therefore, this method is also not suitable for industrial production.

In U.S.Pat.No.7, 271,298, a method for obtaining high content lutein crystals at a high yield by using a simple process is disclosed, wherein the method also does not consider enhancing the proportion of zeaxanthin in the obtained crystals.

In U.S.Pat.No.5,780,693 (CN1178787A), a routine is designed to produce zeaxanthin using lutein as a raw material. In general, dimethyl sulfoxide or the mixture of dimethyl sulfoxide and saturated alkane and/or arene organic solvents are mainly used as solvents, and alkali hydroxides are used as catalysts to produce zeaxanthin through transposition of lutein. Furthermore, the organic solvents, such as n-hexanes, normal heptanes, dichloromethane, methanol and the like, are used during the reaction process. It is obviously improper to use these toxic solvents to produce food grade or medicine grade zeaxanthin.

In U.S.Pat.No.7,485,738, lutein is used as a raw material to obtain high purity meso-zeaxanthin through isomerization with a strong organic base as a catalyst, and the obtained zeaxanthin crystals according to this techinique do not contain or contain very little lutein.

In conclusion, the methods disclosed in the above patents have the following defects:
1) Some toxic organic solvents during the processes, and it is quite difficult or impossible to remove these solvents completely. This results in that the produced lutein or zeaxanthin crystals are not suitable for use in edible products for human beings.
2) The used organic solvents are quite high in viscosity, and thus the operations during the process of separation and purification are quite complicated. In order to obtain higher content crystals, a recrystallation process with several steps is needed, and thus it is not suitable for industrial production.
3) The yield of carotenoid is quite low. Since several steps are involved in the process, the yield is low, which is only about 50%. In particular, when marigold flowers are used as the raw materials to obtain a mixture of zeaxanthin and lutein through isomerization reaction to partially convert the lutein crystals after lutein crystals are obtained through saponification reaction, separation and purification, the yield is even lower.
4) The concentration of the single lutein or zeaxanthin in the product is quite high, and the concentration of the lutein and zeaxanthin in the product may not be adjusted by controlling the reaction process according to the actual needs.

Therefore, it is desired to find a method that is suitable for the industrial large-scale production of high purity lutein and zeaxanthin, in which as little as possible toxic organic solvents and as few as possible operation steps are used but with a high yield, and the concentration of lutein and zeaxanthin in the product may be adjusted by controlling the reaction parameters according to the actual needs.

### SUMMARY

To overcome the defects in the conventional method, the present invention provides a method for the preparation of lutein crystals with a higher content of zeaxanthin from a plant oleoresin, which achieves the objective of conveniently and effectively adjusting the content of the zeaxanthin in the lutein crystals.

To this end, the method according to the present invention comprises the following steps:
a) mixing a lutein diester-containing plant oleoresin with a food-grade alcohol solvent, stirring the mixture to form a homogenous freely flowing mixed solution, and then saponifying the mixed solution for 3 to 5 hours under a basic environment at a temperature from 40 to 85°C;
b) further adding an organic solvent and an emulsifier to the mixed solution obtained in step a), and then stirring the mixed solution uniformly;
c) dropwise adding a strong base solution into the mixed solution obtained in step b), such that a part of the obtained lutein crystals are converted into zeaxanthin in an isomerization manner, and adjusting the reaction time and temperature according to the ratio of lutein to zeaxanthin;
d) diluting the reaction solution obtained in step c) by using a mixed solvent formed by deionized water and the alcohol solvent, such that a volume concentration of the solute in the solution is reduced to 10 to 15%, the amounts of the deionized water and the alcohol solvent being respectively 2 to 10 times and 0.5 to 2 times of the weight of the raw material plant oleoresin, then heating the solution to 60 to 75°C and slowly stirring the solution for 0.5 to 2 hours such that lutein forms granular crystals through precipitation;
e) obtaining the crystals precipitated in step d) by means of centrifugation or filtration;
f) rinsing the obtained crystals obtained in step e) for 2 to 3 times by using 60 to 85°C hot water, until the rinsed mother solution is approximately colorless; and
g) for ease of drying the final product, before drying, washing the filter cake by using an absolute ethanol one time for crystallization, and then obtaining the lutein crystals by means of vacuum drying or freeze drying, having a final drying weight loss being less than 5%.

According to the present invention, the plant oleoresin is an extract from marigold flowers, calendulas, spinaches, strawberries, broccolis, cabbages or corns. In these starting materials, the marigold flower extract contains a relatively higher content of lutein, and is thus a preferable starting material. Based on the species, growing conditions, cropping periods and extraction methods, the oleoresin contains 5 to 30% of lutein diester and other trace carotenoids, for example, the all-trans zeaxanthin, alpha- and beta-cryptoxanthin, and beta-carotene.

By the ultraviolet-visible spectrophotometry analysis and the HPLC analysis, it is known that the final product contains 75 to 95% of carotenoid, which contains 15 to 95% of all-trans luteins, 85 to 5% of zeaxanthin, 0.1 to 1.0% of other possible geometric isomers, as well as less than 1.0% of other carotenoids. There is completely no need to worry about the harm of the other trace carotenoids, because they are also food sources, and their concentrations in human blood serum are much higher as compared with the lutein.

In the final crystallization product prepared by using this process, there is no residual of a toxic organic solvent and other toxic compounds, and the product is thus suitable to be used as food of human.

In step a) of the present invention, generally 1 part by weight of plant oleoresin is dissolved in 0.5 to 2 volume times of the food-grade alcohol solvent, and through stirring, the lutein ester and the other impurities such as wax, resin, and the other carotenoids and pigments are dissolved or dispersed in the solvent to form a homogeneous solution. A base solution (for example, NaOH, KOH, sodium methoxide or sodium ethoxide) is slowly dropwise added into the homogeneously dispersed solution for saponification, and a desired amount of the base for saponification is 0.5 to 6 times the mass of the lutein diester in the plant oleoresin. During the isomerization reaction, the lutein, zeaxanthin and other carotenoids are dissociated, and meanwhile the fatty acid (for example, myristic acid, palmitic acid, stearic acid, or the like) in the plant oleoresin forms soda soap or potash soap with the sodium ions or potassium respectively, and the used food-grade alcohol solvent may be methanol, ethanol, isopropanol, isobutyl acetate, preferably ethanol.

In the step b) and c) of the invention, a specific amount of organic solvent is further added to enhance the solubility of the dissociated carotenoid in the solution, the amount of the organic solvents is 1.0 to 3.0 volume times the weight of the same plant oleoresin, and the organic solvent may be an alcohol solvent, such as ethanol, isopropanol, propanol, propylene glycol, or the like. Further, the organic solvent may also be an ester solvent, such as ethyl acetate, ethyl ester isobutyl acetate or the like; or may be another other organic solvent, such as dimethyl sulfoxide, methylene chloride, or the like. When a specific amount of emulsifier is added, the carotenoid may be better dissolved. The used emulsifier may be a Tween emulsifier, for example, Tween-60 or the like; or may be a Span emulsifier, for example, Span-40 or the like. The amount of the emulsifier is 0.4-1.0 time the weight of lutein crystals in plant oleoresins, in this way, after a specific amount of strong base (the amount of strong base is 0.1-1.0 time the weight of lutein diester in the plant oleoresin) is dropwise added, there is no need to perform additional steps to separate the lutein crystals, and a part of lutein may be directly converted into zeaxanthin in an isomerization manner. The ratio of lutein to zeaxanthin in the reaction product may be monitored by means of high performance liquid chromatography on the sample. The reaction time and temperature may be adjusted according to the ratio of lutein to zeaxanthin, the reaction time is within the range of 0.5 to 6 hours, and the temperature is within the range of 60 to 90°C.

In step e) of the present invention, the generated crystals generated in step d) are separated by means of a traditional separation process, such as centrifugation, filtration, press filtration or the like. Before the separation, to further reduce the concentration of the crystals such that the subsequent separation operation is simple to carry out, the solution may be diluted with a suitable amount of hot water. Through the separation operation, such impurities as fatty acid, salt, soap, water-solubility chlorophyll and flavone may be transferred to the mother solution, and such crystals as the lutein and zeaxanthin remain in the filter cake.

It should be noted that the purity of the final product, the ratio of lutein to zeaxanthin and the yield of the carotenoid crystals not only depend on the amount of the added solvent during the saponification process and the amount of the added alcohol solvent during the dilution process, as well as the amount of dropwise added base, the reaction time and the reaction temperature during the isomerization reaction, but also depend on the crystallization time before washing.

If the proportion of the solvent in the reaction is too small, the viscosity of the solution is high, which is unfavorable to the progress of the reaction and the subsequent separation operation. If the proportion of the solvent in the reaction is too large, the viscosity of the lutein ester in the solution is low, which is unfavorable to complete saponification, reduces the yield of the product, and lowers the content of the crystals. This is the reason why a part of the solvent needs to be further added before the isomerization reaction. The oleoresin has a relative great solubility in the used solution, whereas the carotenoid is obtained upon completion of the saponification reaction. Therefore, the solubility of these crystals in the organic solvent is significantly lowered. However, to convert these crystals to the zeaxanthin, the crystals need to be dissolved to participate in the reaction. Therefore, upon completion of the saponification reaction and before the isomerization reaction, a specific amount of organic solvent needs to be further added. In this way, in one aspect, the concentration of the oleoresin before the saponification reaction may not be lowered, and the carotenoid oleoresin may be saponified within a short period of time, thereby improving the content of carotenoid in the final product; and in another aspect, the reactant, that is, the free carotenoid crystals, during the isomerization reaction may be ensured to be sufficiently dissolved, thereby making the isomerization reaction controllable.

It is very important to add in batches the base in different reaction stages. The free carotenoid crystals are obtained through saponification of the plant oleoresin. However, before these crystals are dissolved in the organic solvent to trigger the isomerization reaction, the concentration of the base shall not be too high; otherwise, a part of the carotenoid oleoresin and the resulted carotenoid crystals may be subjected to oxidation due to high concentration base, or even subjected to carbonization. By adding the base in different amounts in different stages may prevent such phenomena. In the subsequent process of isomerizing the lutein to generate the zeaxanthin, dropwise addition is also to prevent such phenomena.

The addition of a specific amount of emulsifier before the isomerization reaction is also favorable to the isomerization reaction. This is because in the saponification reaction and the isomerization reaction, a specific amount of base solution needs to be added as a catalyst of the reaction, and a small amount of water is inevitably introduced. Presence of the water definitely greatly reduces the solubility of the carotenoid crystals in the organic solvent, thereby reducing the probability of the isomerization reaction. Further, by adding a specific amount of emulsifier, the impact caused by the added water to the solubility of the carotenoid crystals in the organic solvent may be reduced to the minimum, which greatly improves the solubility of the carotenoid crystals and is thus favorable to the reaction. In addition, presence of a small amount of emulsifier also increases the chance that the base catalyst is in contact with the carotenoid crystals, thereby facilitating progress of the reaction.

Furthermore, the refining process of the alcohol solution before the separation of the carotenoid crystals also causes impacts on the content and yield of the product. During the dilution process, if the alcohols are excessively added, the yield of the product may be lowered; and if the alcohols are insufficiently added, the operation difficulty may be increased. The precipitation and crystallization duration shall not be two short, and otherwise, the yield may be lowered.

The present invention has the following advantages:
1) The plant oleoresin is used as a starting material, crystals containing lutein and zeaxanthin are obtained by consecutive two steps of reactions, that is, the saponification reaction and the isomerization reaction. The ratio of the lutein to zeaxanthin is adjusted by controlling the reaction conditions according to the actual needs, which facilitates subsequent product application and effectively prevents inconvenience in purchase, storage and operation caused due to respectively mixture of the lutein and zeaxanthin during the product application.
2) Through a series of condition optimizations, a crystal mixture of the lutein and zeaxanthin may be obtained at a high yield. During the reaction, no additional operation is needed to purify the lutein crystals and the isomerization reaction may be directly carried out. As such, this attains a higher yield as compared with the current process in which high purity lutein crystals are obtained first and then the crystal mixture of the lutein and zeaxanthin is obtained by partially isomerizing the lutein.
3) By adding the organic solvent and the base catalyst in batches, the normal occurrence of the reaction is protected, the reaction duration is shorted as much as possible and a complete reaction is ensured, and damages caused by the strong base to the reaction product and the base materials are mitigated.
4) In the stage of the isomerization reaction, by adding a specific amount of emulsifier, the solubility of the carotenoid crystals is increased, and the chance that the base catalyst is in contact with the reaction base materials is effectively increased, thereby achieving a more sufficient reaction.
5) Since the solvent used in the process is water and a low alcohol or an ester which has a low viscosity, the subsequent separation operation is relatively simpler, and no additional process of recrystallization and refining of the organic solvent is needed. In conclusion, the method according to the present invention is economic and is suitable for large-scale commercialized production.

The present invention is further described with reference to specific examples.

### DETAILED DESCRIPTION

### Example 1

1000 g of marigold oleoresin (a total amount of lutein is 15.2%) was mixed with 2000 mL of isopropanol, the mixture was heated to 40°C, the mixture was then stirred to form a homogenous freely flowing mixed solution, and then 405 ml of 45% NaOH solution was slowly dropwise added for 60 minutes while stirring, and the saponification reaction was carried out for 5 hours at this temperature.

The reaction solution was heated to 60°C, 2400 mL of isopropanol solution and 80 g of Tween-80 emulsifier were added, and the solution was then stirred for 0.5 hour to make it uniformly mixed. Afterwards, 240 mL of 37% CH₃ONa was slowly dropwise added for 1 hour, meanwhile the solution was heated to 70°C, and the reaction was carried out for 0.5 hour while stirring.

Upon completion of the reaction, 2000 mL of deionized water and 200 mL of ethanol were added, the solution temperature was maintained at 60°C during the dilution process, and the solution was slowly stirred for 30 minutes. The obtained crystals were separated by means of centrifugation, and this separation process was relatively simpler and was completed within 10 minutes. The collected crystals were washed with 60°C hot water for 2 to 3 times until the elute is colorless, and the crystals were then washed with an absolute ethanol and then were dried at 40°C in a vacuum environment until the drying weight loss is less than 5%.

Finally, 144.6 g of finished product was obtained, which contains 87.2% of total carotenoid (analyzed by using an ultraviolet-visible spectrophotometer). The carotenoid contains 95.1% of all-trans lutein, 4.8% of all-trans zeaxanthin (analyzed by using HPLC), and the balance of other trace carotenoids. The yield of the carotenoid was 82.95%.

The resulted product did not contain any toxic organic solvent, and is suitable for use in the forms of nutritional supplements and food additives. The application form of this crystal may be oil suspension (emulsified through mixing with a plant oil), beadlet (microcapsule obtained by atomizing condensation), dry powder (microcapsules obtained by spray-drying), or the like.

### Example 2

500 g of marigold oleoresin (a total content of lutein is 14.5%) was mixed with 250 mL of propanol, the mixture was heated to 85°C, the mixture was then stirred to form a homogenous freely flowing mixed solution, and then 210 mL of 50% KOH solution was slowly dropwise added for 60 minutes while stirring, and the saponification reaction was carried out for 3 hours at this temperature.

The temperature of the reaction solution was lowered to 60°C, 1500 mL of ethyl acetate solution and 65 g of Span-40 emulsifier were added, and the solution was then stirred for 0.5 hour to make it uniformly mixed. Afterwards, 100 mL of 50% sodium ethoxide solution was slowly dropwise added for 1 hour, meanwhile the solution was heated to 80°C, and the reaction was carried out for 6 hours while stirring.

Upon completion of the reaction, 1000 mL of deionized water and 250 mL of ethanol were added, the solution temperature was maintained at 60°C during the dilution process, and the solution was slowly stirred for 120 minutes. The obtained crystals were separated by means of filtration, and this separation process was relatively simpler and was completed within 30 minutes. The collected crystals were washed with 85°C hot water for 2 to 3 times until the elute is colorless, and the crystals were then washed with an absolute ethanol and then were dried at 40°C in a vacuum environment until the drying weight loss is less than 5%.

Finally, 61.0 g of finished product was obtained, which contains 85.6% of total carotenoid (analyzed by using an ultraviolet-visible spectrophotometer). The carotenoid contains 15.2% of all-trans lutein, 84.8% of all-trans zeaxanthin (analyzed by using HPLC), and the balance of other trace carotenoids. The yield of the carotenoid was 72.02%.

The resulted product did not contain any toxic organic solvent, and is suitable for use in the forms of nutritional supplements and food additives. The application form of this crystal may be oil suspension (emulsified through mixing with a plant oil), beadlet (microcapsule obtained by atomizing condensation), dry powder (microcapsules obtained by spray-drying), or the like.

### Example 3

1000 g of marigold oleoresin (a total content of lutein is 14.5%) was mixed with 2500 mL of ethanol, the mixture was heated to 75°C, the mixture was then stirred to form a homogenous freely flowing mixed solution, and then 380 mL of 50% KOH solution was slowly dropwise added for 60 minutes while stirring, and the saponification reaction was carried out for 4 hours at this temperature.

The reaction solution was heated to 60°C, 1200 mL of propanol solution and 60 g of Tween-60 emulsifier were added, and the solution was then stirred for 0.5 hour to make it uniformly mixed. Afterwards, 100 mL of 37% sodium methoxide was slowly dropwise added for 1 hour, meanwhile the solution was heated to 80°C, and the reaction was carried out for 3 hours while stirring.

Upon completion of the reaction, 1000 mL of deionized water and 250 mL of ethanol were added, the solution temperature was maintained at 60°C during the dilution process, and the solution was slowly stirred for 120 minutes. The obtained crystals were separated by means of press filtration, and this separation process was relatively simpler and was completed within 30 minutes. The collected crystals were washed with 85°C hot water for 2 to 3 times until the elute is colorless, and the crystals were then washed with an absolute ethanol and then were dried at 40°C in a vacuum environment until the drying weight loss is less than 5%.

Finally, 141.6 g of finished product was obtained, which contains 84.7% of total carotenoid (analyzed by using an ultraviolet-visible spectrophotometer). The carotenoid contains 87.1% of all-trans lutein, 12.8% of all-trans zeaxanthin (analyzed by using HPLC), and the balance of other trace carotenoids. The yield of the lutein was 82.71%.

### Examples 4-7

| Items/Steps | Example 4 | Example 5 | Example 6 | Example 7 |
|---|---|---|---|---|
| Amount of the raw material (g) and the lutein content thereof | 300 g/14.5% | 1500 g/13.6% | 480 g/15.4% | 600 g/14.6% |
| Type and amount of the solvent during the saponification reaction | methanol/600 ml | ethanol/200 ml | propanol/500 ml | ethanol/800 ml |
| Type and amount of the base catalyst during the saponification reaction | 37% sodium methylate solution/118 ml | 50% sodium ethoxide solution / 816ml | 50% KOH ethanol solution/1780 ml | 45% NaOH solution/400 ml |
| Type and amount of solvent further added before the isomerization reaction | Ethyl acetate/300 ml | ethanol/ 1500 ml | isopropanol/1200 ml | isobutyl acetate/1800 ml |
| The types and quantities of emulsifiers | Tween-60/17.5 g | Span-40/204 g | Tween-80/52.0 g | Span-20/88.0 g |
| Type and amount of dropped-add base liquid before the isomerization reaction | 50% sodium ethoxide solution/87 ml | 50% KOH ethyl alcohol solution/820 ml | 45% NaOH solution/330 ml | 37% sodium methylate solution/50 ml |
| Temperature of the isomerization reaction | 90°C | 60°C | 70°C | 85°C |
| Duration of the isomerization reaction | 3.0 hours | 1.0 hours | 4.5 hours | 6.0 hours |
| Quantity of the final product (g) | 42.9g | 178.8g | 71.5g | 72.5g |
| Total carotenoid content (%, UV) in the final product | 76.4% | 82.3% | 74.8% | 88.4% |
| Ratio of lutein to zeaxanthin in the final product (HPLC) | 94.6%/5.2% | 85.2%/14.5% | 30.4%/69.4% | 15.2%/84.7% |

### Comparative Example

1000 g of marigold oleoresin (a total content of the lutein is 14.5%) was saponified according to the process conditions disclosed in US Patent No. 7,271,298, and impurities were removed by using an aqueous alcohol solution to obtain 129.6 g of carotenoid, having a content of 93.2%, the product yield was 83.3%, wherein the all-trans lutein accounted for 93.1% and the all-trans zeaxanthin accounted for 6.8%.

120 g of the lutein crystals were weighed and subjected to an isomerization reaction according to the method disclosed in US Patent No. 7,485,738, the reaction degree was monitored by using the HPLC chromatography, and the reaction was stopped 1.5 hours later. The impurities were further removed by using the aqueous alcohol solution, and finally 73.1 g of carotenoid was obtained, having a content of 85.6%, wherein the all-trans lutein accounted for 88.1%, the all-trans zeaxanthin accounted for 11.8%, and the yield of the isomerization reaction was 54.6%.

The total yield in the above two steps of reactions was only 45.5%, which was far less than the product yield according to the present invention.

## Claims

1. A method for the preparation of lutein crystals with a higher content of zeaxanthin from a plant oleoresin, comprising the following steps:
a) mixing a lutein diester-containing plant oleoresin with a food-grade alcohol solvent, stirring the mixture to form a homogenous freely flowing mixed solution, and then saponifying the mixed solution for 3 to 5 hours under a basic environment at a temperature from 40 to 85°C;
b) further adding an organic solvent and an emulsifier to the mixed solution obtained in step a), and then stirring the mixed solution uniformly;
c) dropwise adding a strong base solution into the mixed solution obtained in step b), such that a part of the obtained lutein crystals are converted into zeaxanthin in an isomerization manner;
d) diluting the reaction solution obtained in step c) by using a mixed solvent formed by deionized water and the alcohol solvent, such that a volume concentration of the solute in the solution is reduced to 10 to 15%, the amounts of the deionized water and the alcohol solvent being respectively 2 to 10 times and 0.5 to 2 times of the weight of the raw material plant oleoresin, then heating the solution to 60 to 75°C and slowly stirring the solution for 0.5 to 2 hours such that carotenoid forms granular crystals through precipitation;
e) obtaining the crystals precipitated in step d) by means of centrifugation or filtration;
f) rinsing the obtained crystals obtained in step e) for 2 to 3 times by using 60 to 85°C hot water, until the rinsed mother solution is approximately colorless; and
g) before drying, washing the filter cake by using an absolute ethanol one time for crystallization, and then obtaining the lutein crystals by means of vacuum drying or freeze drying, having a final drying weight loss being less than 5%.

2. The method according to claim 1, wherein the plant oleoresin is an extract from marigold flowers, calendulas, spinaches, strawberries, broccolis, cabbages or corns.

3. The method according to claim 1 or 2, wherein in step a), the food-grade alcohol solvent is added 0.5 to 2 volume times the weight of the plant oleoresin.

4. The method according to claim 3, wherein the strong base is NaOH, KOH, sodium methoxide or sodium ethoxide, a desired amount of the base for saponification is 0.5 to 6 times the mass of the lutein diester in the plant oleoresin, and during the isomerization reaction, the amount of the base is 0.1 to 1 time of the mass of the lutein diester in the plant oleoresin.

5. The method according to claim 1, wherein the further added organic solvent is an alcohol solvent or an ester solvent, having an amount of 1.0 to 3.0 volume times of the weight of the plant oleoresin.

6. The method according to claim 5, wherein the further added solvent is ethanol, isopropanol, propanol, ethyl acetate or isobutyl acetate.

7. The method according to claim 1, wherein the emulsifier is Tween emulsifier or a Span emulsifier, having an amount of 0.4 to 1 time the mass of the lutein crystals in the plant oleoresin.

8. The method according to claim 1, wherein during the isomerization reaction, the reaction time is within the range of 0.5 to 6 hours, and the temperature is within the range of 60 to 90°C.

9. The method according to claim 1, wherein the alcohol solvent is methanol, ethanol, isopropanol or propanol.

## Patentansprüche

1. Verfahren zum Bereiten von Xanthophyllkristallen mit höherem Gehalt an Zeaxanthin aus pflanzlichem Oleoresin, welches die folgenden Schritte umfasst:
a) Mischen eines Xanthophyll-Diester haltigen pflanzlichen Oleoresins mit einem Lebensmittelalkohol-Lösungsmittel, Rühren des Gemischs, um eine homogene frei zulaufende Mischlösung auszubilden; und dann Verseifen der Mischlösung für 3 bis 5 Stunden unter einer alkalischen Umgebung bei einer Temperatur zwischen 40°C bis 85°C;
b) des weiteren Hinzugeben von organischen Lösungsmitteln und Emulgatoren zur in Schritt a) gewonnenen Mischlösung;
c) Tropfenweise Hinzugeben einer starken alkalischen Lösung in die Mischlösung, die in Schritt b) gewonnen wird, so dass mittels Isomerisierung ein Teil der gewonnenen Xanthophyllkristalle in Zeaxanthin umgewandelt wird;
d) Verdünnung der Reaktionslösung aus Schritt c) durch Verwendung gemischter Lösungsmittel aus deionisiertem Wasser und alkoholhaltigen Lösungsmitteln, so dass die Volumenkonzentration des gelösten Stoffes in der Lösung auf 10-50% sinkt, wobei die Dosierung von entionisiertem Wasser und den alkoholhaltigen Lösungsmitteln das 2- bis 10-fache bzw. das 0,5- bis 2-fache des Gewichts des Ausgangsstoffes des pflanzlichem Oleoresins sind, und dann Erhitzen der Lösung auf 60 bis 75°C und langsames Umrühren der Lösung für 0,5 bis 2,0 Stunden, so dass Carotinoid durch Abscheidung Grobkristallen ausbildet;
e) Gewinnen der in Schritt d) abgeschiedenen Kristalle mittels Zentrifugieren oder Filtrieren,
f) 2- bis 3-faches Spülen der in Schritt e) erhaltenen Kristalle mit 60 bis 85°C heißem Wasser, bis die Mutterlösung nahezu farblos ist;
g) Waschen des Filterkuchens vor dem Austrocknen unter einmaliger Verwendung reinen Ethanols zur Kristallisation, und dann Gewinnen der Xanthophyllkristalle mittels Vakuumtrocknung oder Gefriertrocknungmit einem endgültigen Trockungsgewichtsverlust von weniger als 5%.

2. Verfahren nach Anspruch 1, wobei das pflanzliche Oleoresin ein Extrakt der Ringelblume, Calendula, Spinat, Erdbeeren, Brokkoli, Kohl oder Mais ist.

3. Verfahren nach Anspruch 1 oder 2, wobei in Schritt a) Lebensmittelalkohol-Lösungsmittel um das 0,5- bis 2,0-fache des Gewichts an pflanzlichen Oleoresinen zugegeben werden.

4. Verfahren nach Anspruch 3, wobei die starken Alkalien NaOH, KOH, Natriummethoxid oder Natriumethoxid sind, wobei das erforderliche Volumen an Alkalien das 0,5- bis 6,0-fache der Masse an Xanthophylldiester im pflanzenhaltigen Oleoresine ist, und wobei während der Isomerisierungsreaktion das Volumen an Alkalien das 0,1- bis 1,0-fache der Masse an Xanthophylldiester im pflanzenhaltigen Oleoresin ist.

5. Verfahren nach Anspruch 1, wobei die weiter hinzugegebenen organischen Lösungsmittel Alkohollösungsmittel oder Esterlösungsmittel sind, welche eine Dosierung des 1,0- bis 3,0-fachen Volumens des Gewichts an pflanzlichen Oleoresinen haben.

6. Verfahren nach Anspruch 5, wobei die weiter hinzugegebenen Lösungsmittel Ethanol, Isopropylalkohol, Propanol, Ethylacetat oder Essigsäureisobutylester sind.

7. Verfahren nach Anspruch 1, wobei der Emulgator Tween-Emulgator oder Span-Emulgator ist, welcher eine Dosierung des 0,4- bis 1,0-fachen des Gewichts von Xanthophyllkristallen in pflanzlichen Oleoresinen hat.

8. Verfahren nach Anspruch 1, wobei während der Isomerisierungsreaktion die Reaktionszeit 0,5 bis 6,0 Stunden beträgt und der Temperaturbereich bei 60 bis 90°C liegt.

9. Verfahren des Anspruchs 1, wobei die Alkohollösungsmittel Methanol, Ethanol, Isopropylalkohol oder Propanol sind.

## Revendications

1. Un procédé destiné à la préparation de cristauxde lutéine comportant une teneur plus élevée de zéaxanthine provenant d'une plante oléorésine, comprenant les étapes suivantes :
a) mélanger un diester de lutéine contenant la plante oléorésine comportant un f solvant d'alcool de qualité alimentaire, remuer le mélange pour former une solution mixte homogène circulant librement et ensuite saponifier la solution mixte pendant 3 à 5 heures sous un environnement basique à une température de 40 à 85 °C ;
b) ajouter en outre un solvant organique et un émulsifiant à la solution mixte obtenue dans l'étape a) et ensuite, agiter la solution mixte uniformément ;
c) ajouter goutte à goutte une solution basique forte dans la solution mixte obtenue dans l'étape b) de sorte qu'une partie des cristaux de lutéine obtenus sont convertis en zéaxanthine dans une manière d'isomérisation ;
d) diluer la solution de réaction obtenue dans l'étape c) à l'aide d'un solvant mixte formé par de l'eau désionisée et le solvant d'alcool, de sorte qu'une concentration en volume de soluté dans la solution est réduite de 10 à 15 %, les quantités d'eau désionisée et de solvant d'alcool étant respectivement de 2 à 10 fois et de 0,5 à 2 fois le poids de la matière première de la plante oléorésine, ensuite chauffer la solution à 60 à 75 °C et agiter lentement la solution pendant 0,5 à 2 heures de sorte que le caroténoïdeforme des cristaux granulaires par précipitation ;
e) obtenir les cristaux précipités dans l'étape d) par un moyen de centrifugation ou de filtration ;
f) rincer les cristaux obtenus dans l'étape e) de 2 à 3 fois à l'aide d'eau chaude de 60 à 85 °C, jusqu'à ce que la solution mère rincée soit approximativement incolore ; et
g) avant le séchage, laver le gâteau de filtration à l'aide d'éthanol absolu une fois pour la cristallisation et ensuite pour obtenir les cristaux de lutéineau moyen du séchage sous vide ou par lyophilisation, présentant une perte de poids de séchage final qui est inférieure à 5 %.

2. Le procédé selon la revendication 1, dans lequel la plante oléorésine est un extrait de fleurs de tagètes, de calendulas, d'épinards, de fraises, de brocolis, de choux ou de maïs

3. Le procédé selon la revendication 1 ou 2, dans lequel dans l'étape a) au solvant d'alcool de qualité alimentaire est ajouté un volume de 0,5 à 2 fois le poids de la plante oléorésine.

4. Le procédé selon la revendication 3, dans lequel la base forte est NaOH, KOH, méthylate de sodium ou éthylate de sodium, une quantité souhaitée de la base destinée à la saponification est de 0,5 à 6 fois la masse du diester de lutéine dans la plante oléorésine et, lors de la réaction d'isomérisation, la quantité de la base est de 0,1 à 1 fois la masse de diester de lutéine dans la plante oléorésine.

5. Le procédé selon la revendication 1, dans lequel le solvant organique ajouté en outre est un solvant d'alcool ou un solvant d'ester, présentant une quantité de 1.0 à 3.0 volumes fois le poids de la plante oléorésine.

6. Le procédé selon la revendication 5, dans lequel le solvant ajouté en outre est parmi éthanol, isopropanol, propanol, acétate d'éthyle ou acétate d'isobutyle.

7. Le procédé selon la revendication 1, dans lequel l'émulsifiant est un émulsifiant Tween ou un émulsifiant Span, présentant une quantité de 0,4 à 1 fois la masse des cristaux de lutéine dans la plante oléorésine.

8. Le procédé selon la revendication 1, dans lequel lors de la réaction d'isomérisation, le temps de réaction est dans la plage de 0,5 à 6 heures et la température est dans la plage de 60 à 90 °C.

9. Le procédé selon la revendication 1, dans lequel le solvant d'alcool est parmi méthanol, éthanol, isopropanol ou propanol.
